# EUROPÄISCHE PATENTSCHRIFT

(11) **EP 0 777 457 B1**
(45) Veröffentlichungstag und Bekanntmachung des Hinweises auf die Patenterteilung: **07.08.2002**
(21) Anmeldenummer: 96915016.8
(22) Anmeldetag: 03.05.1996
(51) Int. Cl.: A61K 7/043

(54) **DIE VERWENDUNG VON GLYCERYLTRIACETAT ZUR BEHANDLUNG VON ONYCHOMYKOSEN**
USE OF GLYCERYL TRIACETATE FOR TREATING ONYCHOMYCOSES
UTILISATION DU TRIACETATE DE GLYCERYLE POUR LE TRAITEMENT D'ONYCHOMYCOSES

(30) Priorität: 18.05.1995 DE 19518262
(43) Veröffentlichungstag der Anmeldung: 11.06.1997
(73) Patentinhaber: HOECHST AKTIENGESELLSCHAFT, 65929 Frankfurt am Main (DE)
(72) Erfinder: BOHN, Manfred, D-65719 Hofheim (DE); KRAEMER, Karl, D-63225 Langen (DE); MARKUS, Astrid, D-65835 Liederbach (DE)
(86) Internationale Anmeldenummer: EP9601855
(87) Internationale Veröffentlichungsnummer: WO9636311

(56) Entgegenhaltungen:
- EP-A- 0 226 984
- EP-A- 0 389 778
- EP-A- 0 565 072
- "Martindale- The extra pharmacopoeia" 1993 , THE PHARMACEUTICAL PRESS , LONDON XP002011008 30 in der Anmeldung erwähnt siehe "Triacetin"
- DATABASE MEDLINE AN:94349782, Juni 1994 XP002011009 & CURTIS, Bd. 53, Nr. 6, Juni 1994, Seiten 313-316, MONTANA ET AL.:

## Beschreibung

Pilzerkrankungen der Nägel (Onychomykosen) gehören zu den Infektionskrankheiten, die bis heute nicht befriedigend zu therapieren sind. Obwohl sie für den Patienten weitgehend schmerzfrei verlaufen, macht das permanente Vorhandensein eines Infektionsherdes eine Therapie unbedingt erforderlich. Als prädisponierende Faktoren für Nagelpilzerkrankungen kommen neben Durchblutungsstörungen und Traumatisierungen auch Immunabwehrdefekte sowie bestimmte Stoffwechselstörungen (Diabetes mellitus) in Frage. Bevorzugt befallen werden Fußnägel, wobei hauptsächlich die Nägel der großen und kleinen Fußzehe betroffen sind.

Die Analyse des Erregerspektrums zeigt, daß in erster Linie Dermatophyten (z.B. Trichophyton rubrum), aber auch Hefe (z.B. Candida albicans) oder Schimmelpilze (z.B. Scopulariopsis brevicaulis) Onychomykosen hervorrufen. Abgesehen von der weißen, superfiziellen Onychomykose, spielt sich die Mehrzahl der Infektionen unterhalb der Nagelplatte, im Nagelbett oder im Bereich der Matrix ab. In Abhängigkeit vom klinischen Erscheinungsbild sowie dem Schweregrad der Infektion werden systemische, lokale oder kombinierte Therapieformen diskutiert. Die für den Patienten schonenste und nebenwirkungsärmste Art der Behandlung besteht in einer Lokaltherapie des erkrankten Nagels.

Eine bekannte Behandlungsform ist ein antimykotisch wirksames Präparat in Form eines Nagellacks, welches mindestens eine antimykotisch wirksame Substanz und mindestens einen wasserunlöslichen Filmbildner enthält (EP O 389 778). Als Nachteil dieser Art von Zubereitungen hat sich die geringe laterale Diffusion der zum Einsatz kommenden antimykotisch wirksamen Substanzen im Nagel erwiesen, so daß eine effektive Behandlung der befallenen Nagelmatrix und der unter dem Nagelwall liegenden Areale des Nagelbettes nur sehr unvollkommen möglich ist.

Glyceryltriacetat (1,2,3-Propantriyl-triacetat; C₉ H₁₄ O₆) findet in erster Linie Anwendung als Weichmacher für Lackzubereitungen und ist zu diesem Zwecke auch in einer Zubereitung gemäß EP 0 389 778 enthalten. Darüber hinaus besitzt Glyceryltriacetat fungistatische Eigenschaften und wird deshalb gelegentlich, insbesondere in den USA, zur Behandlung von oberflächlichen Hautpilzerkrankungen verwendet, aber nicht für Onychomykosen (Martindale "The Extra Pharmacopoeia", 30th Edition 1993; Roche Lexikon Medizin 3. Auflage, 1993)

Es wurde nun gefunden, daß sich Glyceryltriacetat hervorragend zur Behandlung von Onychomykosen eignet, weil es eine gute laterale Diffusion durch den Nagel aufweist und deshalb die Behandlung und Prophylaxe der durch Dermatophyten befallenen Nagelmatrix und der unter dem Nagelwall liegenden Areale des Nagelbettes ermöglicht.

Die Erfindung betrifft einen antimykotisch wirksamen Nagellack, enthaltend
a) Glyceryltriacetat,
b) ein 1-Hydroxy-2-pyridon der Formel I 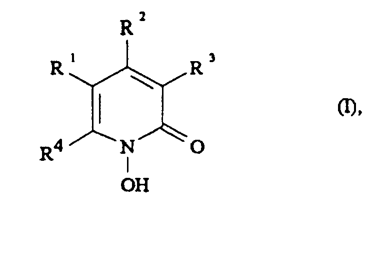 worin R¹, R² und R³, die gleich oder verschieden sind, Wasserstoffatom oder Alkyl mit 1 - 4 Kohlenstoffatomen bedeuten, und
   R⁴ einen gesättigten Kohlenwasserstoffrest mit 6 bis 9 Kohlenstoffatomen oder einen Rest der Formel II bedeutet 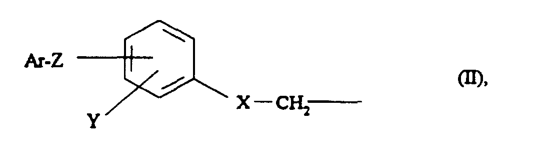 wobei
   - X: S oder O bedeutet,
   - Y: Wasserstoffatom oder bis zu 2 Halogenatome wie Chlor und/oder Brom bedeutet,
   - Z: eine Einfachbindung oder die zweiwertigen Reste O, S, -CR²-(R = H oder C₁-C₄-Alkyl) oder andere zweiwertige Reste mit 2 - 10 kettenförmig verknüpften C- und gegebenenfalls O- und/oder S-Atomen, wobei - wenn die Reste 2 oder mehr O- und/oder S-Atome enthalten - letztere durch mindestens 2 C-Atome voneinander getrennt sein müssen und wobei 2 benachbarte C-Atome auch durch eine Doppelbindung miteinander verknüpft sein können und die freien Valenzen der C-Atome durch H und/oder C₁-C₄-Alkylgruppen abgesättigt sind, bedeutet,
   - Ar: ein aromatisches Ringsystem mit bis zu zwei Ringen, das durch bis zu drei Reste aus der Gruppe Fluor, Chlor, Brom, Methoxy, C₁-C₄-Alkyl, Trifluormethyl und Trifluormethoxy substituiert sein kann, bedeutet, und
c) einen wasserunlöslichen Filmbildner.

Der Begriff "gesättigt" bezeichnet hierbei solche Reste, die keine aliphatischen Mehrfachbindungen, also keine äthylenischen oder actetylenischen Bindungen enthalten.

In den Resten "Z" sind die C-Kettenglieder vorzugsweise CH₂-Gruppen. Wenn die CH₂-Gruppen durch C₁-C₄-Alkylgruppen substituiert sind, sind CH₃ und C₂H₅ bevorzugte Substituenten. Beispielhafte Reste "Z" sind: -O-, -S-, -CH₂-, -(CH₂)ₘ- (m = 2 - 10), -C(CH₃)₂-, -CH₂O-, -OCH₂-, -CH₂S-, -SCH₂-, -SCH(C₂H₅)-, -CH = CH-CH₂O-, -O-CH₂-CH = CH-CH₂O-, -OCH₂-CH₂O-, -OCH₂-CH₂CH₂O-, -SCH₂CH₂CH₂S-, -SCH₂CH₂CH₂CH₂O-, -SCH₂CH₂OCH₂CH₂O-, -SCH₂CH₂OCH₂CH₂O-CH₂CH₂S- oder -S-CH₂-C(CH₃)₂-CH₂-S-.

Der Rest "S" bedeutet Schwefelatom, der Rest "O" bedeutet Sauerstoffatom. Der Begriff "Ar" bedeutet Phenyl und kondensierte Systeme wie Naphthyl, Tetrahydronaphthyl und Indenyl, sowie isolierte Systeme wie solche, die sich vom Biphenyl, Diphenylalkanen, Diphenylethern und Diphenylthioethern ableiten.

In der Formel I ist der Kohlenwasserstoff-Rest R⁴ ein Alkyl- oder Cyclohexylrest, der auch über eine Methylen- oder Äthylengruppe an den Pyridonring gebunden sein oder einen Endomethylgruppe enthalten kann. R⁴ kann auch einen aromatischen Rest darstellen, der jedoch vorzugsweise über wenigstens ein aliphatisches C-Atom an den Pyridonrest gebunden ist.

Die Erfindung betrifft ferner die Verwendung dieses Nagellades zur Herstellung eines Arzneimittels zur Behandlung und Prophylaxe von Onychomykosen.

Mit dem erfindungsgemäßen Arzneimittel läßt sich insbesondere bei den Behandlungen von Nagelmatrixmykosen eine durchgreifende Heilung erzielen, wobei der Nagel gewöhnlich ohne Deformierung nachwächst, weil die Dermatophyten in der Nagelwurzel am Wachstum gehindert werden. Im Hinblick auf die bisherigen schlechten Therapieerfahrungen ist dies ein überaus wichtiger Befund.

Das erfindungsgemäße Arzneimittel eignet sich auch zur prophylaktischen Anwendung gegen Nagelmykosen, wobei ein ausreichend hohes Wirkstoffdepot im Nagel erreicht wird, so daß es im Falle einer Pilzkontamination nicht zum Ausbruch einer durch Pilze hervorgerufenen Nagelerkrankung kommt.
Der Gehalt an Glyceryltriacetat in dem erfindungsgemäßen Arzneimittel beträgt im allgemeinen von 0,1 bis 25 Gewichtsprozent (Gew.-%), vorzugsweise von 2 bis 10 Gew.-%. Der Mindestgehalt von Glyceryltriacetat beträgt mindestens 0,1 bis 1 Gew.-% im Arzneimittel; das zur Prophylaxe verwendete Arzneimittel enthält weniger als 2 und zweckmäßig mindestens 1 Gew.-% Glyceryltriacetat.

In dem erfindungsgemäßen Nagellack, also der Lösemittel enthaltenden Zubereitung, ist Glyceryltriacetat, bezogen auf die Menge der flüchtigen und nicht flüchtigen Bestandteile, im allgemeinen in einer Menge von 1 bis 25 Gew.-%, vorzugsweise 2 bis 10 Gew.-% enthalten. Der Gehalt von Glyceryltriacetat in den medizinischen Nagellacken, also denen zur Behandlung, beträgt meistens 2 Gew.-%; die zur Prophylaxe verwendeten Nagellacke enthalten meistens weniger als 2 Gew.-% und zweckmäßig mindestens 1 Gew.-% Glyceryltriacetat. In den erfindungsgemäßen Nagellacken ist Glyceryltriacetat im allgemeinen in einer Menge von 6 bis 80 Gew.-%, vorzugsweise von 10 bis 70 Gew.-% und insbesondere von 15 bis 60 Gew.-% enthalten, jeweils bezogen auf die Menge der nicht flüchtigen Bestandteile, das ist die Summe der Filmbildner, der gegebenenfalls vorhandenen Pigmente, Weichmacher und anderen nicht flüchtigen Zusätze.

Die erfindungsgemäßen Nagellacke enthalten außer dem in einem Lösemittel oder Lösemittelgemisch gelösten Glyceryltriacetat als notwendige Bestandteile noch einen oder mehrere Filmbildner, die nach dem Trocknen der Zubereitung einen wasserunlöslichen Film auf dem Nagel bilden.

Als wasserunlösliche Filmbildner eignen sich beispielsweise Stoffe auf der Basis von Cellulosenitrat oder physiologisch unbedenkliche Polymerisate wie sie in Kosmetika üblich sind, vorzugsweise als Gemisch mit Cellulosenitrat. Genannt seien beispielsweise Polyvinylacetat und partiell verseiftes Polyvinylacetat, Mischpolymerisate aus Vinylacetat einerseits und Acrylsäure oder Crotonsäure oder Maleinsäuremonoalkylester andererseits, ternäre Mischpolymerisate aus Vinylacetat einerseits und Crotonsäure und Vinylneodecanoat, oder Crotonsäure und Vinylpropionat andererseits, Mischpolymerisate aus Methylvinylether und Maleinsäuremonoalkylester, insbesondere als Maleinsäuremonobutylester, Mischpolymerisate aus Fettsäurevinylester und Acrylsäure oder Methacrylsäure, Mischpolymerisate aus N-Vinylpyrrolidon, Methacrylsäure und Methacrylsäurealkylester, Mischpolymerisate aus Acrylsäure und Methacrylsäure oder Acrylsäurealkylester oder Methacrylsäurealkylester, insbesondere mit einem Gehalt an quartären Ammoniumgruppen, oder Polymere, Copolymere oder Mischungen, enthaltend Ethylacrylat, Methylmethacrylat oder Trimethylammonioethylmethacrylat-chlorid, oder Polyvinylacetale und Polyvinylbutyrale, alkylsubstituierte Poly-N-vinylpyrrolidone, Alkylester aus Mischpolymerisaten aus Olefinen und Maleinsäureanhydrid und Umsetzungsprodukte von Kolophonium mit Acrylsäure. In den Estern sind die Alkylreste gewöhnlich kurzkettig und haben meistens nicht mehr als vier C-Atome.

Als physiologisch unbedenkliche Lösemittel kommen Stoffe wie in Kosmetika übliche Kohlenwasserstoffe, halogenierte Kohlenwasserstoffe, Alkohole, Äther, Ketone und Ester in Betracht, insbesondere Essigsäureester von einwertigen Alkoholen, wie Äthyl- und Butylacetat, gegebenenfalls im Gemisch mit aromatischen Kohlenwasserstoffen, wie Toluol, und/oder Alkoholen, wie Äthanol oder Isopropanol. Die Kombination der Lösemittel ist bekanntlich von entscheidender Bedeutung für die Trockenzeit, Verstreichbarkeit und andere wichtige Eigenschaften des Lackes oder des Lackfilms. Das Lösemittelsystem besteht vorzugsweise aus einer optimalen Mischung aus Niedrigsiedern (= Lösemittel mit einem Siedepunkt bis 100 °C) und Mittelsiedern (= Lösemittel mit einem Siedepunkt bis 200 °C).

Die erfindungsgemäßen Nagellacke können weiterhin in Kosmetika gebräuchliche Zusätze enthalten wie Weichmacher auf Phthalat- oder Campherbasis, Farbstoffe oder Farbpigmente, Perlglanzmittel, Sedimentationsverzögerer, Suflonamidharze, Silikate, Riechstoffe, Netzmittel wie Natriumdioctylsulfosuccinat, Lanolinderivate, Lichtschutzmittel wie 2-Hydroxy-4-methoxybenzophenon, antibakteriell wirksame Substanzen und Stoffe mit keratolytischer und/oder keratoplastischer Wirkung wie Ammoniumsulfit, Ester und Salze der Thioglykolsäure, Harnstoff, Allantoin, Enzyme und Salizylsäure.

Gefärbte oder pigmentierte Nagellacke haben beispielsweise den Vorteil, daß die erfindungsgemäße Zubereitung dem Schönheitsempfinden des Patienten angepaßt werden kann.

Die Herstellung des erfindungsgemäßen Arzneimittels und des Nagellacks erfolgt in üblicher Weise durch Zusammengeben der einzelnen Komponenten und eine - soweit erforderlich - der jeweiligen Zubereitung angepaßte Weiterverarbeitung.

Wichtige Vertreter der durch die Formel I charakterisierten Verbindungsklasse sind:
6-[4-(4-Chlor-phenoxy)-phenoxymethyl]-1-hydroxy-4-methyl-2-pyridon, 6-[4-(2,4-Dichlor-phenoxy)-phenoxymethyl]-1-hydroxy-4-methyl-2-pyridon, 6-(Biphenylyl-4-oxy-methyl)-1-hydroxy-4-methyl-2-pyridon, 6-(4-Benzylphenoxymethyl)-1-hydroxy-4-methyl-2-pyridon, 6-[4-(2,4-Dichlorbenzyloxy)-phenoxy-methyl]-1-hydroxy-4-methyl-2-pyridon, 6-[4-(4-Chlor-phenoxy)-phenoxymethyl]-1-hydroxy-3,4-dimethyl-2-pyridon, 6-[4-(2,4-Dichlor-benzyl)-phenoxymethyl]-1-hydroxy-3,4-dimethyl-2-pyridon, 6-[4-(Cinnamyloxy)-phenoxymethyl]-1-hydroxy-4-methyl-2-pyridon, 1-Hydroxy-4-methyl-6-[4-(4-trifluormethyl-phenoxy)-phenoxymethyl]-2-pyridon, 1-Hydroxy-4-methyl-6-cyclohexyl-2-pyridon, 1-Hydroxy-4-methyl-6-(2,4,4-trimethylpentyl)-2-pyridon, 1-Hydroxy-4-methyl-6-n-hexyl-, -6-iso-hexyl-, -6-n-heptyl- oder -6-iso-heptyl-2-pyridon, 1-Hydroxy-4-methyl-6-octyl- oder -6-iso-octyl-2-pyridon, insbesondere 1-Hydroxy-4-methyl-6-cyclohexylmethyl- oder -6-cyclohexyläthyl-2-pyridon, wobei der Cyclohexylrest jeweils auch einen Methylrest tragen kann, 1-Hydroxy-4-methyl-6-(2-bicyclo[2,2,1]heptyl)-2-pyridon, 1-Hydroxy-3,4-dimethyl-6-benzyl- oder -6-dimethylbenzyl-2-pyridon oder 1-Hydroxy-4-methyl-6-(β-phenyl-äthyl)-2-pyridon.

Der Gehalt an der Verbindung der Formel I in dem erfindungsgemäßen Nagellack ist von der Struktur einer jeden Verbindung der Formel I und damit von deren Freigabe aus dem Lackfilm, seinem Penetrationsverhalten im Nagel und seinen antimikrobiellen Eigenschaften abhängig.

In dem erfindungsgemäßen Nagellack, also der Lösemittel enthaltenden Anwendungsform, ist die Verbindung der Formel I, bezogen auf die Menge der flüchtigen und nicht flüchtigen Bestandteile, im allgemeinen in einer Menge von 0,5 bis 20, vorzugsweise 2 bis 15 Gew.-% enthalten. Der Gehalt in den medizinischen Nagellacken, also denen zur Behandlung, beträgt meistens 4 Gew.-%; die zur Prophylaxe verwendeten Nagellacke enthalten meistens weniger als 4 und zweckmäßig mindestens 1 Gew.-% der Verbindung der Formel I. Die Verbindung der Formel I ist im allgemeinen in einer Menge von 2 bis 80 Gewichtsprozent, vorzugsweise von 10 bis 60 Gew.-% und insbesondere von 20 bis 40 Gew.-% in den erfindungsgemäßen Nagellacken enthalten, jeweils bezogen auf die Menge der nicht flüchtigen Bestandteile, das ist die Summe der Filmbildner, Glyceryltriacetat, der gegebenenfalls vorhandenen Pigmente, Weichmacher und anderen nicht flüchtigen Zusätze.

Die Herstellung der Nagellacke, enthaltend eine Verbindung der Formel I, erfolgt durch Zusammengeben der einzelnen Komponenten und eine - soweit erforderlich - der jeweiligen Zubereitungsart angepaßten Weiterverarbeitung (EP 0 226 984). Einige dieser vielfältigen möglichen Zubereitungsformen werden in den Ausführungsbeispielen beispielhaft beschrieben.

### Beispiel 1

Eine erfindungsgemäße Zubereitung weist folgende Zusammensetzung auf:

| | |
|---|---|
| Glyceryltriacetat | 6,0 Gew.-% |
| Isopropylalkohol | 47,0 Gew.-% |
| Äthylacetat | 32,0 Gew.-% |
| Mischpolymerisat aus Methylvinyläther und Maleinsäuremonobutylester | 15,0 Gew.-% |

Der Nagellack wird durch Lösen der verschiedenen Komponenten in den Lösemitteln hergestellt.

### Beispiel 2

Eine erfindungsgemäße Zubereitung weist folgende Zusammensetzung auf:

| | |
|---|---|
| Glyceryltriacetat | 2,5 Gew.-% |
| 1-Hydroxy-4-methyl-6-Cyclohexyl-2-pyridon | 5,0 Gew.-% |
| Isopropylalkohol | 46,5 Gew.-% |
| Äthylacetat | 36,0 Gew.-% |
| Mischpolymerisat aus Methylvinyläther und Maleinsäuremonobutylester | 10,0 Gew.-% |

### Beispiel 3

Eine erfindungsgemäße Zubereitung weist folgende Zusammensetzung auf:

| | |
|---|---|
| Glyceryltriacetat | 2,5 Gew.-% |
| 1-Hydroxy-4-methyl-6-(2,4,4-trimethylpentyl)- 2-pyridon | 2,5 Gew.-% |
| Isopropylalkohol | 47,5 Gew.-% |
| Äthylacetat | 40,0 Gew.-% |
| Mischpolymerisat aus Methylvinyläther und Maleinsäuremonobutylester | 7,5 Gew.-% |

### Beispiel 4

### Wirksamkeitsprüfung

Die Versuche zum Nachweis der lateralen Penetration von Glyceryltriacetat durch Keratinmaterial wird mit den in den Beispielen 1 bis 3 genannten Zubereitungen wie folgt durchgeführt:

Aus dem verhornten Teil eines Kuhhornes werden etwa 0,5 mm dicke, 1,5 x 3 cm große Plättchen präpariert, die an 5 aufeinander folgenden Tagen täglich auf einer Hälfte einer Seite mit der jeweiligen wirkstoffhaltigen Zubereitung behandelt werden. Anschließend werden die Plättchen in einer feuchten Kammer auf Metallzylindern etwa 0,5 cm über einer Wasseragaroberfläche mit den hälftig behandelten Seiten nach unten fixiert. Die gesamte nach oben gewandte Hornseite wird mit einer Mikrokonidiensuspension von Trichophyton mentagrophytes mehrfach punktförmig beimpft. Anschließend wird die Koloniebildung bei 28°C über 10 Tage beobachtet.

### Ergebnis:

Die 5-malige hälftige Vorbehandlung der Hornrückseite mit den erfindungsgemäßen Formulierungen gemäß der Beispiele 1 bis 3 verhinderte auf der gesamten Oberseite die Auskeimung der Pilzsporen vollständig. Selbst die Inokulationspunkte waren auf dem gesamten Oberseite nach einigen Tagen makroskopisch nicht mehr sichtbar. Daher erstreckt sich die Wirkung der erfindungsgemäßen Formulierung lateral von dem Behandlungsbereich über die gesamte Oberfläche des Hornstücks.

Die Kontrollplättchen, ohne Behandlung mit der erfindungsgemäßen Formulierung, zeigen nach Beimpfung vollständiges Wachstum auf der Hornoberfläche.

Ein Kontrollplättchen, behandelt mit einer Zubereitung, enthaltend die Zubereitung gemäß Beispiel 2 jedoch ohne Glyceryltriacetat, zeigt kein Wachstum direkt oberhalb der behandelten Hornseite, während auf der anderen Hälfte des Plättchens (nicht mit der Zubereitung behandelt) Wachstum des Pilzes zu beobachten ist.

## Patentansprüche

1. Nagellack, enthaltend Glyceryltriacetat,
einen oder mehrere Filmbildner, die nach dem Trocknen der Zubereitung einen wasserunlöslichen Film auf dem Nagel bilden und
ein 1-Hydroxy-2-pyridon der Formel I 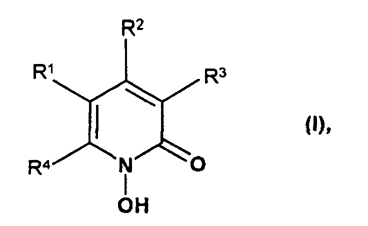 worin R¹, R² und R³, die gleich oder verschieden sind, Wasserstoffatom oder Alkyl mit 1 - 4 Kohlenstoffatomen bedeuten, und
R⁴ einen gesättigten Kohlenwasserstoffrest mit 6 bis 9 Kohlenstoffatomen oder einen Rest der Formel II bedeutet 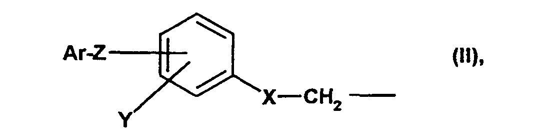 wobei
X S oder O bedeutet,
Y Wasserstoffatom oder bis zu 2 Halogenatome wie Chlor und/oder Brom bedeutet,
Z eine Einfachbindung oder die zweiwertigen Reste O, S, -CR₂- (R = H oder C₁-C₄-Alkyl) oder andere zweiwertige Reste mit 2 - 10 kettenförmig verknüpften C- und gegebenenfalls O- und/oder S-Atomen, wobei - wenn die Reste 2 oder mehr O- und/oder S-Atome enthalten - letztere durch mindestens 2 C-Atome voneinander getrennt sein müssen und wobei 2 benachbarte C-Atome auch durch eine Doppelbindung miteinander verknüpft sein können und die freien Valenzen der C-Atome durch H und/oder C₁-C₄-Alkylgruppen abgesättigt sind, bedeutet und
Ar ein aromatisches Ringsystem mit bis zu zwei Ringen, das durch bis zu drei Reste aus der Gruppe Fluor, Chlor, Brom, Methoxy, C₁-C₄-Alkyl, Trifluormethyl und Trifluormethoxy substituiert sein kann, bedeutet.

2. Nagellack gemäß Anspruch 1, **dadurch gekennzeichnet, daß** man die Verbindung der Formel I einsetzt, worin Ar ein bicyclisches System darstellt, das sich vom Biphenyl, Diphenylalkan oder Diphenylether ableitet.

3. Nagellack gemäß Anspruch 1, **dadurch gekennzeichnet, daß** die Verbindung der Formel I in der Position R⁴ ein Cyclohexylrest enthält.

4. Nagellack gemäß Anspruch 1, **dadurch gekennzeichnet, daß** die Verbindung der Formel I in der Position R⁴ einen Octylrest der Formel -CH₂-CH(CH₃)-CH₂-C(CH₃)₃ enthält.

5. Nagellack gemäß Anspruch 1, **dadurch gekennzeichnet, daß** man 6-[4-(4-Chlorphenoxy)-phenoxymethyl]-1-hydroxy-4-methyl-2-pyridon, 1-Hydroxy-4-methyl-6-cyclohexyl-2-pyridon oder 1-Hydroxy-4-methyl-6-(2,4,4-trimethylpentyl)-2-pyridon einsetzt.

6. Nagellack gemäß Anspruch 1, **dadurch gekennzeichnet, daß** Glyceryltriacetat in einer Menge von 0,1 bis 25 Gewichtsprozent enthalten ist.

7. Nagellack gemäß Anspruch 1, **dadurch gekennzeichnet, daß** Glyceryltriacetat in einer Menge von 1 bis 25 Gewichtsprozent bezogen auf die Menge der flüchtigen und nichtflüchtigen Bestandteile enthalten ist.

8. Nagellack gemäß Anspruch 1, **dadurch gekennzeichnet, daß** als Filmbildner ein Mischpolymerisat aus Methylvinyläther und Maleinsäuremonobutylester eingesetzt wird.

9. Nagellack gemäß einem oder mehreren der Ansprüche 1 bis 5, **dadurch gekennzeichnet, daß** die Verbindung der Formel I in einer Menge von 2 bis 80 Gewichtsprozent, vorzugsweise von 10 bis 60 Gewichtsprozent und insbesondere von 20 bis 40 Gewichtsprozent, bezogen auf die Menge der nicht-flüchtigen Bestandteile enthalten ist.

10. Nagellack gemäß Anspruch 1, **dadurch gekennzeichnet, daß** als Zusatz ein Stoff mit keratolytischer und/oder keratoplastischer Wirkung wie Ammoniumsulfit, Ester und Salze der Thioglykolsäure, Harnstoff, Allantoin, Enzyme und Salizylsäure eingesetzt wird.

11. Verwendung des Nagellacks gemäß der Ansprüche 1 bis 10 zur Herstellung eines Arzneimittels zur Behandlung und Prophylaxe von Onychomykosen

## Claims

1. A nail varnish comprising glyceryl triacetate, one or more film-forming agents which form a water-insoluble film on the nail after the formulation has dried, and a 1-hydroxy-2-pyridone of the formula I 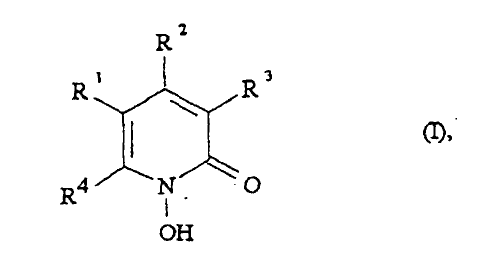 in which R¹, R² and R³, which are.. identical or different, are hydrogen atoms or alkyl having 1 - 4 carbon atoms and
R⁴ is a saturated hydrocarbon radical having 6 to 9 carbon atoms or a radical of the formula II 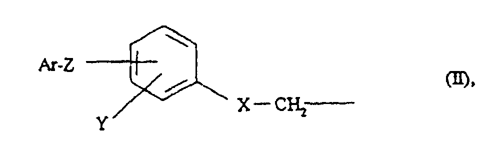 in which
X is S or 0,
Y is a hydrogen atom or up to 2 halogen atoms, such as chlorine and/or bromine,
Z is a single bond or the divalent radicals O, S or -CR₂- (R = H or C₁-C₄-alkyl) or other divalent radicals having 2 - 10 carbon atoms and optionally O and/or S atoms linked in the form of a chain, where - if the radicals contain 2 or more O and/or S atoms- the latter must be separated from one another by at least 2 carbon atoms, and where 2 adjacent carbon atoms can also be linked to one another by a double bond and the free valencies of the carbon atoms are saturated by H and/or C₁-C₄-alkyl groups, and
Ar is an aromatic ring system having up to two rings, which can be substituted by up to three radicals from the group consisting of fluorine, chlorine, bromine, methoxy, C₁-C₄-alkyl, trifluoromethyl and trifluoromethoxy.

2. A nail varnish as claimed in claim 1, wherein the compound of the formula I in which Ar is a bicyclic system derived from biphenyl, diphenylalkane or diphenyl ether is employed.

3. A nail varnish as claimed in claim 1, wherein the compound of the formula I contains a cyclohexyl radical in the position R⁴.

4. A nail varnish as claimed in claim 1, wherein the compound of the formula I contains an octyl radical of the formula -CH₂-CH(CH₃)-CH₂-C(CH₃)₃ in the position R⁴.

5. A nail varnish as claimed in claim 1, wherein 6-[4-(4-chloro-phenoxy)-phenoxymethyl]-1-hydroxy-4-methyl-2-pyridone, 1-hydroxy-4-methyl-6-cyclohexyl-2-pyridone or 1-hydroxy-4-methyl-6-(2,4,4-trimethylpentyl)-2-pyridone is employed.

6. A nail varnish as claimed in claim 1, which comprises glyceryl triacetate in an amount of 0.1 to 25% by weight.

7. A nail varnish as claimed in claim 1, which comprises glyceryl triacetate in an amount of 1 to 25% by weight, based on the amount of volatile and nonvolatile constituents.

8. A nail varnish as claimed in claim 1, wherein a copolymer of methyl vinyl ether and maleic acid monobutyl ester is employed as the film-forming agent.

9. A nail varnish as claimed in one or more of claims 1 to 5, which comprises the compound of the formula I in an amount of 2 to 80% by weight, preferably 10 to 60% by weight and in particular 20 to 40% by weight, based on the amount of nonvolatile constituents.

10. A nail varnish as claimed in claim 1, wherein a substance having a keratolytic and/or keratoplastic action, such as ammonium sulfite, esters and salts of thioglycolic acid, urea, allantoin, enzymes and salicylic acid, is employed as additive.

11. The use of a nail varnish as claimed in any of claims 1 to 10 for the preparation of a medicament for treatment and prophylaxis of onychomycoses.

## Revendications

1. Vernis à ongles contenant du triacétate de glycéryle,
une ou plusieurs substances filmogènes qui, après séchage de la préparation, forment un feuil insoluble dans l'eau sur l'ongle, et
une 1-hydroxy-2-pyridone de formule I 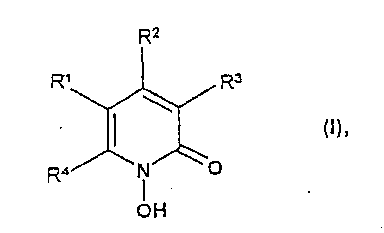 dans laquelle R¹, R² et R³, qui sont identiques ou différents, représentent chacun un atome d'hydrogène ou un groupe alkyle ayant de 1 à 4 atomes de carbone, et
R⁴ est un radical hydrocarboné saturé ayant de 6 à 9 atomes de carbone, ou un radical de formule II 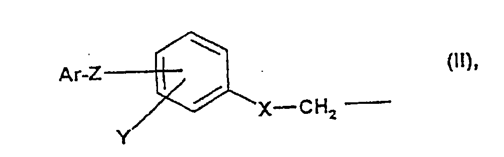 dans laquelle
X est S ou O,
Y est un atome d'hydrogène ou représente jusqu'à 2 atomes d'halogène tels que le chlore et/ou le brome,
Z est une liaison simple, ou les radicaux divalent O, S, -CR²- (R est H ou un groupe alkyle en C₁-C₄) ou d'autres radicaux divalents ayant de 2 à 10 atomes de carbone et éventuellement d'oxygène et/ou de soufre, reliés en chaîne, auquel cas - quand les radicaux contiennent 2 atomes d'oxygène et/ou de soufre, ou plus - ces derniers doivent être séparés l'un de l'autre par au moins 2 atomes de carbone, et où 2 atomes de carbone voisins peuvent aussi être liés l'un à l'autre par une double liaison, et les valences libres des atomes de carbone sont saturées par H et/ou des groupes alkyle en C₁-C₄,
Ar est un système cyclique aromatique ayant jusqu'à deux cycles, qui peut être substitué par jusqu'à trois radicaux choisis dans l'ensemble comprenant les groupes fluoro, chloro, bromo, méthoxy, alkyle en C₁-C₄, trifluorométhyle et trifluorométhoxy.

2. Vernis à ongles selon la revendication 1, **caractérisé en ce qu'**on utilise le composé de formule I dans laquelle Ar est un système bicyclique qui dérive du biphényle, d'un diphénylalcane ou du diphényléther.

3. Vernis à ongles selon la revendication 1, **caractérisé en ce que** le composé de formule I contient sur la position R⁴ un radical cyclohexyle.

4. Vernis à ongles selon la revendication 1, **caractérisé en ce que** le composé de formule I contient sur la position R⁴ un radical octyle de formule -CH₂CH(CH₃)-CH₂-C(CH₃)₃.

5. Vernis à ongles selon la revendication 1, **caractérisé en ce qu'**on utilise la 6-[4-(4-chlorophénoxy)-phénoxyméthyl]-1-hydroxy-4-méthyl-2-pyridone, la 1-hydroxy-4-méthyl-6-cylohexyl-2-pyridone ou la 1-hydroxy-4-méthyl-6-(2,4,4-triméthylpentyl)-2-pyridone.

6. Vernis à ongles selon la revendication 1, **caractérisé en ce que** le triacétate de glycéryle est présent en une quantité de 0,1 à 25 % en poids.

7. Vernis à ongles selon la revendication 1, **caractérisé en ce que** le triacétate de glycéryle est présent en une quantité de 1 à 25 % en poids par rapport à la quantité des constituants volatils et non volatils.

8. Vernis à ongles selon la revendication 1, **caractérisé en ce qu'**on utilise en tant que substance filmogène un copolymère de méthylvinyléther et de maléate de monobutyle.

9. Vernis à ongles selon l'une ou plusieurs des revendications 1 à 5, **caractérisé en ce que** le composé de formule I est présent en une quantité de 2 à 80, de préférence de 10 à 60 et en particulier de 20 à 40 % en poids par rapport à la quantité des constituants non volatils.

10. Vernis à ongles selon la revendication 1, **caractérisé en ce qu'**on utilise en tant qu'additif une substance ayant un effet kératolytique et/ou kératoplastique tel que le sulfite d'ammonium, les esters et sels de l'acide thioglycolique, l'urée, l'allantoïne, les enzymes et l'acide salicylique.

11. Utilisation du vernis à ongles selon les revendications 1 à 10 pour préparer un médicament destiné au traitement et à la prophylaxie des onychomycoses.
